# EUROPÄISCHE PATENTSCHRIFT

(11) **EP 1 967 155 B1**
(45) Veröffentlichungstag und Bekanntmachung des Hinweises auf die Patenterteilung: **01.02.2012**
(21) Anmeldenummer: 08003111.5
(22) Anmeldetag: 20.02.2008
(51) Int. Cl.: A61B 19/00

(54) **Medizinische Klemme, insbesondere Wirbelsäulenklemme**
Medical clamp, in particular spinal clamp
Pince médicale, en particulier pince de colonne vertébrale

(30) Priorität: 08.03.2007 DE 102007011568
(43) Veröffentlichungstag der Anmeldung: 10.09.2008
(73) Patentinhaber: Siemens Aktiengesellschaft, 80333 Munich (DE)
(72) Erfinder: Hüfner, Tobias, Prof., Dr., 30177 Hannover (DE); Dannenmann, Tim, Dr., 96047 Bamberg (DE); Citak, Musa, Dr., 30449 Hannover (DE); Karsak, Özcan, 91074 Herzogenaurach (DE)
(74) Vertreter: Nobis, Wolfgang

(56) Entgegenhaltungen:
- WO-A-00/78233
- WO-A-03/009768
- US-A- 4 834 090
- US-A- 5 795 308
- US-A1- 2004 019 263
- US-A1- 2006 084 867

## Beschreibung

Die Erfindung betrifft eine medizinische Klemme, insbesondere eine Wirbelsäulenklemme.

Bildgeführte Interventionen, insbesondere CT-geführte Interventionen, sind heute Teil der klinischen Routine. Im Gegensatz zu einer invasiven chirurgischen Behandlung ermöglichen dabei minimalinvasive bildgeführte Interventionen dem Anwender eine Arbeit mit minimalen Verletzungen des Patienten. Dies verringert nicht nur die klinischen Kosten. Es verringert auch die Gefahr von Komplikationen und besitzt einen positiven kosmetischen Effekt.

Zur Durchführung solcher bildgeführter Interventionen ist es erforderlich, medizinische Instrumente im Inneren des Körpers des Patienten zu navigieren. Unter dem Begriff Navigation wird dabei das Bestimmen der Position durch Ortung, das Planen des Zugangsweges zum Zielpunkt sowie das Führen eines medizinischen Instrumentes zu diesem Zielpunkt auf dem geplanten Zügangsweg verstanden. Es ist bekannt, zu diesem Zweck an dem Körper des Patienten wenigstens eine Referenzmarkierung ("dynamische Referenz-Basis"; DRB) anzubringen.

Dabei kann es sich beispielsweise um optische Markierungen (bspw. von einer Kamera erfaßbare kugelförmige Reflektoren) oder elektromagnetische Markierungen (bspw. in einem elektromagnetischen Feld anregbare Spulen) handeln. Mit Hilfe der Referenzmarkierung kann dann die Lage des medizinischen Instrumentes relativ zu dem Körper des Patienten bestimmt und damit für die Navigation genutzt werden.

Bisher war es üblich, Referenzmarkierungen auf der Haut des Patienten zu befestigen, beispielsweise aufzukleben. Bei schwierigen Interventionen, insbesondere im Wirbelsäulenbereich, ist diese Art der Positionierung der Referenzmarkierungen jedoch für eine exakte Navigation des medizinischen Instruments zu ungenau. Dies liegt darin begründet, daß sich Bewegungen der Oberfläche des Patientenkörpers nicht ganz vermeiden lassen. Es wird daher dazu übergegangen, Referenzmarkierungen direkt am Knochen des Patienten zu befestigen, beispielsweise durch Nageln oder Schrauben. Bei Interventionen an der Wirbelsäule eines Patienten erfolgte bisher das Anbringen der Referenzmarkierung zumeist mit Hilfe von Klemmen an exponierten Knochen, beispielsweise am Dornfortsatz. Für all diese Methoden sind jedoch vergleichsweise starke Eingriffe in den Patienten erforderlich, weshalb eine solche Positionierung von Referenzmarkierungen aufwendig und riskant ist und darüber hinaus die oben skizzierten Vorteile einer minimalinvasiven Intervention zum großen Teil wieder zunichte machen.

Aus der internationalen Patentanmeldung WO 00/78233 ist eine medizinische Klemme bekannt, welche zwei Klemmschenkel aufweist, die mit einem Griffteil verbunden sind, wobei das Griffteil zur Ausführung einer Klemmbewegung der Klemmschenkel ausgebildet ist, und wobei die Klemmschenkel lösbar mit dem Griffteil verbunden sind.

Aus der US-Patentanmeldung 4, 834, 090 ist eine medizinische, Klemme bekannt, bei der eine flexible Gummihülse, die eine mittig angeordnete Öffnung aufweist, in der Mitte geknickt und auf die Arme der Klemme aufgeschoben werden kann. Die Gummihülse muß zum Freilegen der Arme von der Klemme abgezogen werden.

Eine Aufgabe der vorliegenden Erfindung ist es, eine neuartige medizinische Klemme bereitzustellen, die besonders sichere, minimalinvasive perkutane Interventionen, insbesondere im Bereich der wirbelsäule, ermöglicht.

Diese Aufgabe wird durch eine medizinische Klemme, wie sie nachfolgend im Detail beschrieben ist, gelöst. Bei der Klemme handelt es sich insbesondere um eine Wirbelsäulenklemme mit zwei Klemmschenkeln, die mit einem Griffteil verbunden sind, wobei das Griffteil zur Ausführung einer Klemmbewegung der Klemmschenkel ausgebildet ist.

In der Klemme sind verschiedene Grundgedanken verwirklicht, denen allesamt die Erkenntnis zugrunde liegt, daß an der Verwendung der Klemm-Methode festgehalten werden kann, wenn es möglich ist, eine medizinische Klemme bereitzustellen, die sowohl den Anforderungen einer minimalinvasiven Intervention genügt, als auch dem Gesichtspunkt einer sicheren Positionierung einer Referenzmarkierung Rechnung trägt.

Gemäß einer ersten Grundidee der Erfindung ist die Klemme modular aufgebaut dargestellt, daß die Klemmschenkel lösbar mit dem Griffteil verbunden sind. Dadurch, daß die Klemmschenkel von dem Griffteil gelöst werden können, ist es möglich, verschiedene Klemmschenkel zu verwenden, insbesondere solche Klemmschenkel, die für den einzelnen Anwendungsfall, d.h. für die konkrete Anatomie bestmöglich geeignet sind. Durch den modularen Aufbau der Klemme verringern sich die Anschaffungskosten, da das Griffteil nur jeweils einmal angeschafft werden muß. Anstelle von vielen verschiedenen medizinischen Klemmen müssen lediglich ein einziges Griffstück und die benötigten Klemmschenkel angeschafft werden.

Eine weitere Grundidee der Erfindung ist es, individuell an die besondere Anatomie des Anwendungsortes (z.B. des Wirbelkörpers) angepaßte Klemmschenkel einzusetzen. Dabei finden insbesondere Klemmschenkel Verwendung, die L-förmige oder Y-förmige Klemmbacken aufweisen.

Die Y-förmigen Klemmbacken werden vorzugsweise für eine besonders sichere, rotationsstabile Fixierung im Bereich der Lendenwirbelsäule verwendet. Dort werden sie am Dornfortsatz befestigt. Durch die Y-Form wird die Klemmkraft auf eine größere Fläche verteilt, die derart ausgebildet ist, daß ein zusätzlicher Schutz gegen ein Verdrehen der Klemme entsteht.

Die L-förmigen Klemmbacken eignen sich besonders für den Bereich der oberen Brustwirbelsäule, da dort die Dornfortsätze schmaler sind und horizontal verlaufen.

Eine weitere Grundidee der Erfindung ist es, bei der Verwendung von geraden Klemmbacken eine Abdeckvorrichtung zu verwenden, welche die oft mit Spitzen oder Kanten versehenen Klemmbacken während des Einführens der Klemme in den Körper des Patienten abdeckt. Dadurch werden unnötige Verletzungen des Gewebes vermieden.

Weitere Vorteile ergeben sich aus den bevorzugten Ausführungsformen der Erfindung, die in den Unteransprüchen angegeben und/oder in den nachfolgend anhand der Zeichnungen näher erläuterten Ausführungsbeispielen beschrieben sind. Hierbei zeigen:
- Fig. 1: eine medizinische Klemme mit einer Referenzmarkierung zur Verwendung für ein Positionierungssystem für perkutane Interventionen,
- Fig. 2: einen Klemmschenkel mit einer geraden Klemmbacke, wie in Fig. 1 abgebildet, in einer ersten und einer zweiten Seitenansicht,
- Fig. 3: eine Abdeckvorrichtung für einen Klemmschenkel, wie in Fig. 2 abgebildet, in einer ersten und einer zweiten Seitenansicht,
- Fig. 4: einen Klemmschenkel mit einer Y-förmigen Klemmbacke in einer ersten und einer zweiten Seitenansicht,
- Fig. 5: einen Klemmschenkel mit einer weiteren Y-förmigen Klemmbacke in einer Seitenansicht,
- Fig. 6: einen Klemmschenkel mit einer L-förmigen Klemmbacke in einer Seitenansicht,
- Fig. 7: einen Klemmdorn in einer Seitenansicht und einer Draufsicht.

Die Figuren zeigen die Erfindung lediglich schematisch und mit ihren wesentlichen Bestandteilen. Sämtliche Maße sind in Millimeter angegeben. Die angegebenen Maße sind ausschließlich als für die hier gezeigten Beispiele maßgeblich anzusehen und dienen lediglich Illustrationszwecken. Alle angegebenen Maße können im Rahmen der Erfindung abgeändert werden.

Die erfindungsgemäße Wirbelsäulenklemme 1 umfaßt zwei Klemmschenkel 2, die mit einem Griffteil 3 verbunden sind, wobei das Griffteil 3 zur Ausführung einer Klemmbewegung der Klemmschenkel 2 ausgebildet ist. Zu diesem Zweck weist das Griffteil 3 einen ersten Betätigungsschenkel 4 und einen zweiten Betätigungsschenkel 5 auf, die relativ zueinander beweglich sind. Die beiden Betätigungsschenkel 4, 5 sind an ihrem einen Ende über ein Schwenkgelenk 6 nach Art eines Zirkels miteinander verbunden. Diese stabile und einfache Konstruktion gewährleistet eine hohe Ausfallsicherheit.

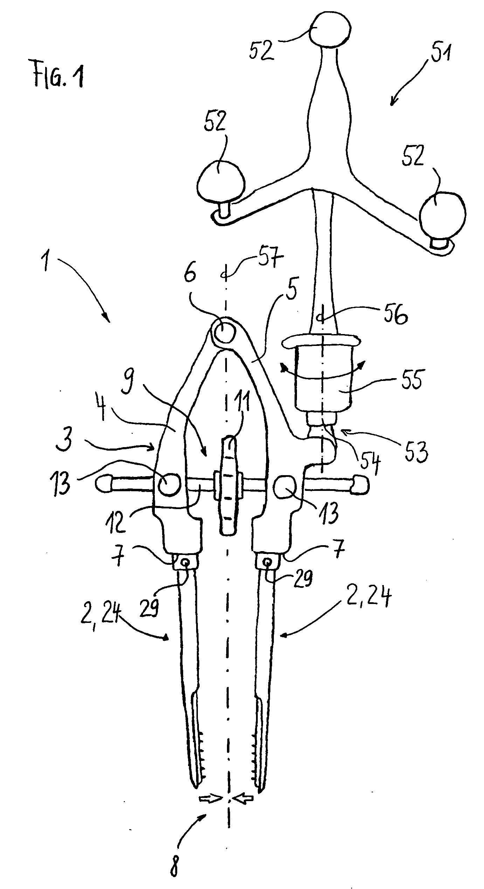

Die den Verbindungsenden gegenüberliegenden Stirnflächen 7 des Griffteils 3 sind in einer geöffneten Stellung der Klemme 1, wie in Fig. 1 dargestellt, voneinander beabstandet. Dadurch ergibt sich auch ein vorgegebener Abstand der Klemmschenkel 2, welcher es erlaubt, die Klemmschenkel 2 über zwei voneinander beabstandete Hautschnitte in den Körper eines Patienten einzuführen. Die Hautschnitte müssen dabei lediglich so bemessen sein, daß sie die Klemmschenkel 2 aufnehmen können. Ein Einführen des Griffteils 3 in den Körper ist nicht notwendig.

Zum Schließen und Öffnen des Klemmenmauls 8 sind die beiden Betätigungsschenkel 4, 5 über einen Mitteltrieb 9 aufeinander zu und voneinander weg bewegbar. Der Mitteltrieb 9 umfaßt eine mit einer Rändelscheibe 11 versehene Spindel 12 mit einem Gewinde und ist vorzugsweise selbsthemmend ausgeführt. Somit ist bei einer Befestigung der Klemme 1 an einem Knochen eine besonders feste und sichere Fixierung möglich, ohne daß sich die Klemme 1 selbsttätig lösen kann. Auf die in den Betätigungsschenkeln 4, 5 vorzusehenden Elemente 13 zur Aufnahme der Gewindespindel 12 wird hier nicht näher eingegangen; technische Lösungen hierzu sind dem Fachmann bekannt. Alternativ zu der Rändelscheibe kann auch ein sternförmiges Rad verwendet werden.

In Fig. 1 ist eine Stellung abgebildet, bei der die beiden Klemmschenkel 2 bereits etwas aus der Normalstellung, in der die beiden Klemmschenkel 2 parallel zueinander stehen, heraus und aufeinander zu bewegt sind. Die Klemmschenkel 2 sind einzeln lösbar mit dem Griffteil 3 verbunden. Somit können die Klemmschenkel 2 auch einzeln, beispielsweise im Fall eines Defektes oder dergleichen bzw. wenn es die anatomische Situation erfordert, ausgetauscht werden. Die Verbindung zwischen den Klemmschenkeln 2 und dem Griffteil 3 wird über Schnapp-, Rast- oder Klemmverbindungen hergestellt, wobei sich entsprechende Aufnahme- bzw. Verbindungsöffnungen (nicht abgebildet) in den unteren Stirnflächen 7 der Betätigungsschenkel befinden. Dadurch ist eine einfache und dennoch sehr sichere Verbindung realisierbar. In den Zeichnungen sind evtl. an den Klemmschenkeln 2 erforderliche Befestigungselemente nicht abgebildet. Auf die zur Ausbildung einer geeigneten Befestigung der Klemmschenkel 2 an dem Griffteil 3 notwendigen Mittel wird nachfolgend nicht weiter eingegangen.

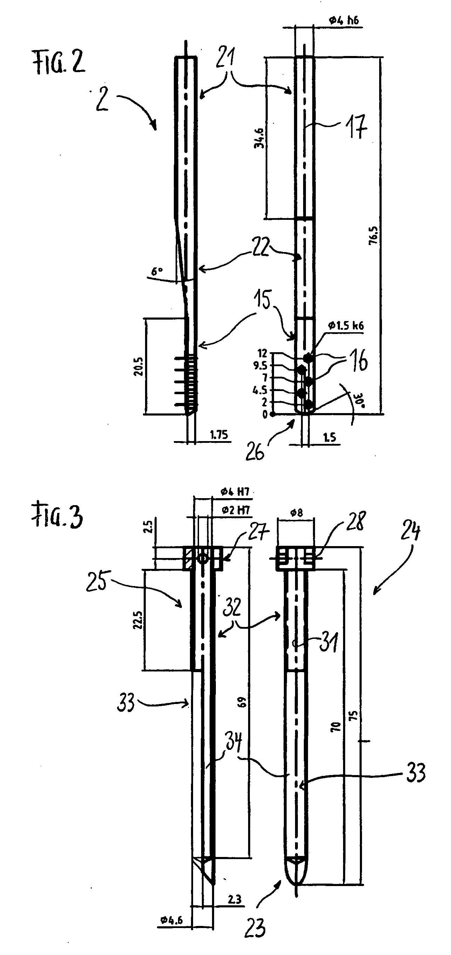

Ein Klemmschenkel 2, wie er in Fig. 1 dargestellt ist, weist eine gerade, also parallel zur Mittellängsachse 17 des Klemmschenkels 2 verlaufende Klemmbacke 15 auf, vgl. auch Fig. 2. Die Klemmbacke 15 weist eine Länge von 20,5 mm auf und ist mit Fixierdornen 16 versehen, die zu der Mittellängslinie 17 des 76,5 mm langen Klemmschenkels um jeweils 1,5 mm versetzt angeordnet sind, um eine bessere Fixierung am Knochengewebe zu erreichen. Die genaue Positionierung der Fixierdorne 16 ergibt sich aus den in Fig. 2 angegebenen Abstandsangaben.

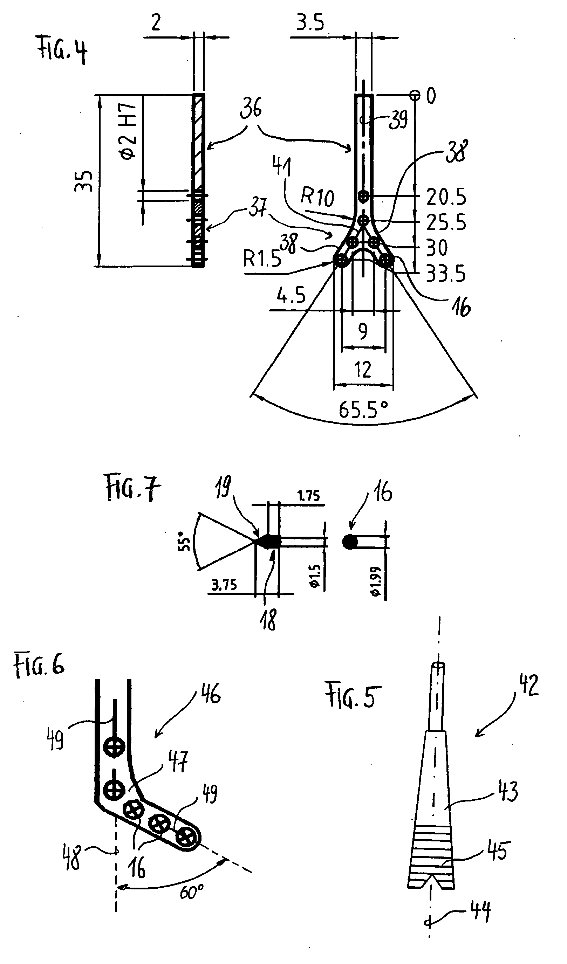

Ein Beispiel eines solchen Fixierdorns 16 ist in Fig. 7 abgebildet. Ein Fixierdorn 16 besteht vorzugsweise aus einem zylindrischen Schaft 18 und einer an dem Schaft 18 befestigten kegelförmigen Spitze 19. Die Spitze 19 weist an der Verbindungsstelle mit dem Schaft 18 einen größeren Durchmesser (hier 1,99 mm) auf als der Schaft (hier 1,5 mm) und bildet daher eine Art Widerhaken aus. Die Gesamtlänge des Fixierdorns 16 beträgt in dem gezeigten Beispiel 3,75 mm, bei einer Schaftlänge von 1,75 mm. Bei einem Anziehen der Klemme 1 bohren sich die Fixierdorne 16 in den zu operierenden Knochen und verhindern ein Abrutschen der Klemme 1.

Die Dicke des Klemmschenkels 2 im Bereich der Klemmbacke 15 ist geringer als die Dicke seines Befestigungsbereiches 21. Durch diese Verjüngung wird die Hautbrücke zwischen den beiden Hautschnitten bei der Anwendung der Klemme 1 geschont. Die Dicke des Klemmschenkels 2 verringert sich dabei in Richtung der Klemmbacke 15 in einem Übergangsbereich 22 kontinuierlich. Im Bereich der Klemmbacke 15 beträgt die Dicke des Klemmschenkels 2 nur noch 1,75 mm. Dies verhindert unnötige Verletzungen des Gewebes beim Eindringen der Klemmschenkel 2 in den Körper des Patienten. Der Übergangsbereich 22 verbindet den proximalen Befestigungsbereich 21 des Klemmschenkels 2, der eine Länge von 34,6 mm aufweist, mit der distal angeordneten Klemmbacke 15.

Besonders vorteilhaft für eine minimalinvasive Anwendung der Klemme 1 ist die Verwendung einer Abdeckvorrichtung 24, wie sie in Fig. 3 dargestellt ist, als Gewebeschutz während des Einführens der Klemmschenkel 2 in den Patientenkörper. Die insgesamt 75 mm lange Abdeckvorrichtung 24 ist als zylinderförmige, nach einer Seite hin teilweise offene Hülse 25 ausgebildet, die von dem distalen Freiende 26 des Klemmschenkels 2 her über den Klemmschenkel 2 geschoben ist. Dabei ist jedem Klemmschenkel 2 eine Abdeckvorrichtung 24 zugeordnet.

Am proximalen Ende weist die Abdeckvorrichtung 24 einen ringförmigen Anschlag 27 auf. Die Anschläge 27 dienen zum einen dazu, eine im Hinblick auf ihrer Verschiebbarkeit in Längsrichtung exakt definierte Lage der Abdeckvorrichtungen 24 relativ zu den Klemmschenkeln 2 herzustellen. Zugleich dienen die Anschläge 27 während des Anziehens der Klemme 1 am Knochen zum Anzeigen der genauen (vorzugsweise identischen) Position beider Hülsen 25 an den Stirnflächen 7 des Griffstücks 3.

An dem Anschlag 27 sind seitlich Öffnungen 28 zur Aufnahme eines Betätigungsstiftes 29 vorgesehen. Der im montierten Zustand senkrecht zur Mittellängsachse 31 der Hülse 25 angeordnete Stift 29 dient als Betätigungselement zum Drehen der Hülse 25 um ihre Mittellängsachse 31. In der in Fig. 1 dargestellten offenen Position der Hülse 25 ragen die Stifte 29 aus der Blattebene in Richtung des Betrachters heraus. Unterhalb des 5 mm breiten Anschlags 27 schließt sich ein erster, 22,5 mm langer geschlossener Hülsenteil 32 an. Im Anschluß daran ist die Hülse 25 durch den Wegfall einer Hülsenhälfte geöffnet. Durch diese Öffnung 33 der Hülse 25 ergibt sich ein durch die verbleibende Hülsenhälfte gebildetes, 47,5 mm langes wannenförmiges Schild 34. Dieses bildet auch das distale Hülsenende 23 und ist dort zur Vermeidung unnötiger Gewebebeschädigungen beim Einführen des Klemmschenkels 2 halbkreisförmig ausgebildet.

Die Abmessungen von Klemmschenkel 2 und Abdeckvorrichtung 24 sind derart aufeinander abgestimmt, daß ein Verdrehen der Hülse 25 um den Klemmschenkel 2 in montiertem Zustand problemlos möglich ist.

Die beiden an einer Klemme 1 vorgesehenen Abdeckvorrichtungen 24 sind auch unabhängig voneinander betätigbar, d.h. sie können unabhängig voneinander von einer ersten Stellung, in der sie die Klemmbacke 15 eines Klemmschenkels 2 vorzugsweise vollständig abdecken, in eine zweite Stellung, in der sie die Klemmbacke 15 des Klemmschenkels 2 nicht abdecken, überführt werden.

Das Prinzip der Abdeckvorrichtung ist nicht auf Klemmschenkel 2 mit geraden Klemmbacken 15 beschränkt. Auch Klemmschenkel mit anders geformten Klemmbacken können hierdurch gesichert werden.

Anstelle der mit einer Abdeckvorrichtung 24 versehenen Klemmschenkel 2 können mit dem in Fig. 1 dargestellten Griffteil 3 auch weitere Klemmschenkel verwendet werden. Beispielhaft ist in Fig. 4 ein 35 mm langer Klemmschenkel 36 mit einer Y-förmigen Klemmbacke 37 abgebildet. Die Klemmarme 38 der Y-förmigen Klemmbacke 37 sind symmetrisch zur Mittellängsachse 39 des Klemmschenkels 36 abgewinkelt und weisen an ihrem distalen Ende einen maximale Breite von 12 mm auf. Die dort vorgesehenen Fixierdorne 16 sind dann voneinander 9 mm beabstandet. Die Klemmarme 38 schließen zwischen sich vorzugsweise einen Winkel von etwa 55° bis 75°. Als besonders vorteilhaft für eine sichere Fixierung hat sich ein Winkel von etwa 60° bis 70° erwiesen. Der in Fig. 4 abgebildete Klemmschenkel 36 weist Fixierdorne 16 auf, die entlang der Mittellinie(n) 41 der Klemmbacke 37 bzw. der Klemmarme 38 angeordnet sind. Die genauen Abmessungen dieses Klemmschenkels 36 sowie die Positionierung der Fixierdorne 16 ergeben sich aus den in Fig. 4 angegebenen Maßangaben.

Fig. 5 zeigt einen weiteren Klemmschenkel 42 mit einer Y-förmigen Klemmbacke 43, welcher sich neben der breiteren Klemmbackenform dadurch auszeichnet, daß anstelle der Fixierdorne schräg aus der Oberfläche der Klemmbacke 43 nach außen ragende, rechtwinklig zur Mittellängsachse 44 des Klemmschenkels 42 verlaufende Fixierkanten 45 bzw. -zacken vorgesehen sind.

Alternativ ist es möglich, Klemmschenkel 46 mit L-förmigen Klemmbacken 47 einzusetzen, vgl. Fig. 6. Diese schließen mit der Mittellängsachse 48 der Klemmschenkel 46 vorzugsweise einen Winkel von etwa 20° bis 90° ein. Als besonders vorteilhaft für eine sichere Fixierung hat sich ein Winkel von etwa 40° bis 70° erwiesen. Auch hier sind Fixierdorne 16 vorgesehen, die entlang der Mittellinie 49 der Klemmbacke 47 angeordnet sind.

Sowohl das Griffteil 3, als auch die Klemmschenkel 2 bestehen vorzugsweise aus rost- und säurebeständigem Stahl. Werden allerdings größere Teile der Klemme 1 aus einem Röntgendurchlässigen Material gefertigt, kann die Anzahl der Metallartefakte im bildgebenden Verfahren verringert werden. Bevorzugte Materialien sind Polyetheretherketon, Glasfaser, kohlefaserverstärktes Polyetheretherketon, Karbon und Keramik. Beispielsweise können die Betätigungsschenkel 4, 5 des Griffteils 3 sowie die Klemmschenkel 2 vollständig aus einem der oben genannten Nichtmetalle gefertigt werden. Darüber hinaus bestehen vorzugsweise sämtliche Elemente der Klemme 1 aus einem sterilisierbaren Material, so daß sie nach einer Benutzung wiederverwendet werden können.

Die Klemme 1 gemäß der vorliegenden Erfindung weist eine Referenzmarkierung 51 mit sternförmig angeordneten kugelförmigen Reflektoren 52 auf zur Verwendung für ein optisches Positionierungssystem für perkutane Interventionen. Beispielsweise sind die Reflektoren 52 derart ausgebildet, daß sie sich zur Erfassung mit einer Infrarotkamera eignen. Die Referenzmarkierung 51 ist mit einer Befestigungsvorrichtung 53 an dem Griffteil 3 der Klemme 1 verbunden. Die Befestigungsvorrichtung 53 umfaßt einen Kupplungszapfen 54, auf den ein Kupplungsstück 55 der Referenzmarkierung 51 aufgesteckt werden kann. Die Längsachse 56 des Kupplungszapfens 54 ist vorzugsweise möglichst weit von der Mittellängsachse 57 der Klemme 1, also der Klemmensymmetrieachse, entfernt. Bei einer metallischen Referenzmarkierung 51 werden durch eine solche exzentrische Anordnung Metallartefakte im bildgebenden Verfahren reduziert, da die Referenzmarkierung 51 aus dem OP-Bereich verlagert ist.

Das Kupplungsstück 55 ermöglicht eine Drehung der Referenzmarkierung 51 derart, daß sich die Reflektoren 52 in der für die Navigation gewünschten Position befinden, was wiederum das Auftreten von Metallartefakten reduziert.

Nachfolgend wird ein Anbringen einer erfindungsgemäßen medizinischen Klemme 1 an einem Knochen, beispielsweise an dem Dornfortsatz der Wirbelsäule beschrieben. Als Beispiel dient eine Klemme 1 mit geraden Klemmbacken 15 und einer Abdeckvorrichtung 24. Der Patient wird in der üblichen Bauchlage gelagert. Nach dem sterilen Abwaschen nach chirurgischem Standart wird die Position der benötigten Hautschnitte ermittelt, beispielsweise mit Hilfe eines CT. Die Hautschnitte sind maximal 1 bis 1,5 cm lang und befinden sich direkt oberhalb des Dornfortsatzes. Nach einem stumpfen Präparieren können die Klemmschenkel 2 der Wirbelsäulenklemme 1 durch die Hautschnitte in den Körper des Patienten eingeführt werden.

Vor dem Einführen wird die Abdeckvorrichtung 24 nach innen verdreht, so daß die Klemmbacken 15 abgedeckt sind und sich die Fixierdorne 16 nicht in dem Weichgewebe verhaken und dieses beschädigen. Nach einer Kontrolle der Position der Klemme 1 durch das CT kann die Klemme 1 angezogen werden. Das Schließen des Klemmenmauls 8 erfolgt über die Rändelscheibe 11, die hier als Drehgriff ausgebildet ist.

Nachdem die modulare Klemme 1 minimalinvasiv an dem Knochen des Patienten unverrückbar befestigt wurde, wird die Position der an der Klemme 1 fixierten Referenzmarkierung 51 mit Hilfe eines kontaktlosen Ortungsverfahrens bestimmt und zur Navigation eines chirurgischen Instrumentes oder dergleichen für einen Wirbelsäuleneingriff verwendet. Einzelheiten zum Aufbau und zur Funktionsweise der einzelnen Komponenten eines solchen Positionierungssystems, insbesondere zum Zusammenführen bzw. Abgleichen der Daten aus dem Referenzsystem einerseits und den (CT-) Bilddaten andererseits, sind aus dem Stand der Technik bekannt, so daß hierauf nicht weiter eingegangen werden muß.

Nach Abschluß der Navigation ist durch einfaches Zurückdrehen der Rändelscheibe 11 die Klemmeigenschaft aufzuheben. Die Wunde kann nach dem chirurgischen Standart zugenäht werden.

Alle in der Beschreibung, den nachfolgenden Ansprüchen und der Zeichnung dargestellten Merkmale können sowohl einzeln als auch in beliebiger Kombination miteinander erfindungswesentlich sein. Insbesondere sind die in den Ansprüchen 11 und 12 bzw. 13 und 14 beschriebenen Ausführungsformen auch separat, d.h. unabhängig von den in den übrigen Ansprüchen beschriebenen Ausführungsformen, realisierbar und stellen bereits für sich genommen schutzfähige Erfindungen dar.

### Bezugszeichenliste

- 1: Klemme
- 2: Klemmschenkel
- 3: Griffteil
- 4: Betätigungsschenkel
- 5: Betätigungsschenkel
- 6: Schwenkgelenk
- 7: Stirnfläche
- 8: Klemmenmaul
- 9: Mitteltrieb
- 10: (frei)
- 11: Rändelscheibe
- 12: Gewindespindel
- 13: Aufnahmeelement
- 14: (frei)
- 15: Klemmbacke
- 16: Fixierdorn
- 17: Mittellängsachse
- 18: Schaft
- 19: Spitze
- 20: (frei)
- 21: Befestigungsende
- 22: Übergangsbereich
- 23: Hülsenende
- 24: Abdeckvorrichtung
- 25: Hülse
- 26: Freiende
- 27: Anschlag
- 28: Öffnung
- 29: Betätigungsstift
- 30: (frei)
- 31: Mittellängsachse
- 32: Geschlossener Hülsenteil
- 33: Öffnung
- 34: Schild
- 35: (frei)
- 36: Klemmschenkel
- 37: Klemmbacke
- 38: Klemmarm
- 39: Mittellängsachse
- 40: (frei)
- 41: Mittellinie
- 42: Klemmschenkel
- 43: Klemmbacke
- 44: Mittellängsachse
- 45: Fixierkante
- 46: Klemmschenkel
- 47: Klemmbacke
- 48: Mittellängsachse
- 49: Mittellinie
- 50: (frei)
- 51: Referenzmarkierung
- 52: Reflektor
- 53: Befestigungsvorrichtung
- 54: Kupplungszapfen
- 55: Kupplungsstück
- 56: Längsachse
- 57: Mittellängsachse

## Patentansprüche

1. Medizinische Klemme (1), insbesondere Wirbelsäulenklemme, mit zwei Klemmschenkeln (2), die klemmbacken (15) aufweisen, wobei die Klemmschenkel (2) mit einem Griffteil (3) verbunden sind, wobei das Griffteil (31) zur Ausführung einer Klemmbewegung der Klemmschentkel (2) ausgebildet ist, und wobei die Klemmschenkel (2) Iösbar mit dem Griffteil (3) verbunden sind, **dadurch gekennzeichnet, daß** jedem Klemmschenkel (2) eine Abdeckvorrichtung (24) zum zumindest teilweise Abdecken der Klemmbacken (15) zugeordnet ist, wobei die Abdeckvorrichtung (24) als rohrförmige, nach einer Seite hin zumindest teilweise offene Hülse (25) ausgebildet ist und wobei die an dem Klemmschenkel (2) angeordnete Abdeckvorrichtung (24) von einer ersten Stellung, in der sie die Klemmbacke (15) des Klemmschenkels (2) zumindest teilweise abdeckt, in eine zweite Stellung, in der sie die Klemmbacke (15) des Klemmschenkels (2) nicht abdeckt, überführbar ist.

2. Medizinische Klemme (1) nach Anspruch 1, **dadurch gekennzeichnet, daß** das Griffteil (3) einen ersten Betätigungsschenkel (4) und einen zweiten Betätigungsschenkel (5) aufweist, welche relativ zueinander beweglich sind.

3. Medizinische Klemme (1) nach Anspruch 2, **dadurch gekennzeichnet, daß** die beiden Betätigungsschenkel (4, 5) an ihrem einen Ende gelenkig miteinander verbunden sind.

4. Medizinische Klemme (1) nach Anspruch 2 oder 3, **dadurch gekennzeichnet, daß** die beiden Betätigungsschenkel (4, 5) über einen Mitteltrieb (9) aufeinander zu und voneinander weg bewegbar sind.

5. Medizinische Klemme (1) nach Anspruch 4, **dadurch gekennzeichnet, daß** der Mitteltrieb (9) eine mit einer Rändelscheibe (11) versehene Spindel (12) aufweist.

6. Medizinische Klemme (1) nach einem der Ansprüche 1 bis 5, **dadurch gekennzeichnet, daß** die Klemmschenkel (2) mit dem Griffteil (3) über eine Schlepp-, Rast- oder Klemmverbindung verbunden sind.

7. Medizinische Klemme (1) nach einem der Ansprüche 1 bis 6, **dadurch gekennzeichnet, daß** die Klemmschenkel (2) einzeln lösbar mit dem Griffteil (3) verbunde sind.

8. Medizinische Klemme (1) nach einem der Ansprüche 1 bis 7, **dadurch gekennzeichnet, daß** die Klemmschenkel (2) gerade Klemmbacken (15) aufweisen.

9. Medizinische Klemme (1) nach einem der Ansprüche 1 bis 8, **dadurch gekennzeichnet, daß** die Klemmschenkel (2) im Bereich der Klemmbacken (15) eine geringere Dicke aufweisen als im Bereich ihres Befestigungsendes (21).

10. Medizinische Klemme (1) nach einem der Ansprüche 1 bis 9, **dadurch gekennzeichnet, daß** sich die Dicke der Klemmschenkel (2) in Richtung der Klemmbacken (15) in einem Übergangsbereich (22) kontinuierlich verringert.

11. Medizinische Klemme (1) nach einem der Ansprüche 1 bis 10, **dadurch gekennzeichnet, daß** die Klemmschenkel (46) L-förmige Klemmbacken (47) aufweisen.

12. Medizinische Klemme (1) nach Anspruch 11, **dadurch gekennzeichnet, daß** die L-förmigen Klemmbacken (47) mit der Mittellängsachse (48) der Klemmschenkel (46) einen Winkel von etwa 20° bis 90° einschließen, vorzugsweise jedoch einen Winkel von etwa 40° bis 70°.

13. Medizinische Klemme (1) nach einem der Ansprüche 1 bis 10, **dadurch gekennzeichnet, daß** die Klemmschenkel (36) Y-förmige Klemmbacken (37) aufweisen.

14. Medizinische Klemme (1) nach Anspruch 13, **dadurch gekennzeichnet, daß** die Klemmarme (38) der Y-förmigen Klemmbacken (37) vorzugsweise symmetrisch zu der Mittellängsachse (39) der Klemmschenkel (36) abgewinkelt sind und zwischen sich einen winkel von etwa 55° bis 75° einschließen, vorzugsweise jedoch einen Winkel von etwa 60° bis 70°.

15. Medizinische Klemme (1) nach einem der Ansprüche 1 bis 14, **dadurch gekennzeichnet, daß** die Abdeckvorrichtungen (24) der Klemmschenkel (2) voneinander unabhängig betätigbar sind.

16. Medizinische Klemme (1) nach einem der Ansprüche 1 bis 15, **dadurch gekennzeichnet, daß** die Klemmschenkel (2) Klemmbacken (15) mit Fixierspitzen (16) oder -kanten (45) aufweisen.

17. Medizinische Klemme (1) nach einem der Ansprüche 1 bis 16, **dadurch gekennzeichnet, daß** Teile der Klemme (1) aus einem Nichtmetall bestehen.

18. Medizinische Klemme (1) nach einem der Ansprüche 1 bis 17, **dadurch gekennzeichnet, daß** an dem Griffteil (3) eine Befestigungsvorrichtung (53) für ein Markierungselement (51) vorgesehen ist, welches ausgebildet ist zur Bestimmung seiner Position mit Hilfe eines kontaktlosen Ortungsverfahrens, insbesondere zur Verwendung als Referenzmarkierung für ein Positionierungssystem für perkutane Interventionen.

19. Medizinische Klemme (1) nach Anspruch 18, mit einem solchen Markierungselement (51), **dadurch gekennzeichnet, daß** das Markierungselement (51) entfernt von der Mittellängsachse (57) der Klemme (1) angeordnet ist.

## Claims

1. Medical clamp (1), in particular spinal clamp, having two clamp limbs (2), which feature clamp jaws (15), the clamp limbs (2) being connected to a grip part (3), the grip part (3) being configured to execute a clamping movement of the clamp limbs (2), and the clamp limbs (2) being connected in a releasable fashion to the grip part (3), **characterised in that** a covering device (24) is assigned to each clamp limb (2) to at least partially cover the clamp jaws (15), the covering device (24) being configured as a tubular sleeve (25) being at least partially open to one side and it being possible to move the covering device (24) disposed on the clamp limb (2) from a first position, in which it at least partially covers the clamp jaw (15) of the clamp limb (2), to a second position, in which it does not cover the clamp jaw (15) of the clamp limb (2).

2. Medical clamp (1) according to claim 1,
**characterised in that** the grip part (3) features a first actuating limb (4) and a second actuating limb (5), which can be moved relative to one another.

3. Medical clamp (1) according to claim 2,
**characterised in that** the two actuating limbs (4, 5) are connected to one another in an articulated fashion at their one end.

4. Medical clamp (1) according to claim 2 or 3,
**characterised in that** the two actuating limbs (4, 5) can be moved towards and away from one another by way of a central drive (9).

5. Medical clamp (1) according to claim 4,
**characterised in that** the central drive (9) features a spindle (12) provided with a knurled disc (11).

6. Medical clamp (1) according to one of claims 1 to 5,
**characterised in that** the clamp limbs (2) are connected to the grip part (3) by way of a snap-fitting, locking or clamping connection.

7. Medical clamp (1) according to one of claims 1 to 6,
**characterised in that** the clamp limbs (2) are connected in an individually releasable fashion to the grip part (3).

8. Medical clamp (1) according to one of claims 1 to 7,
**characterised in that** the clamp limbs (2) feature straight clamp jaws (15).

9. Medical clamp (1) according to one of claims 1 to 8,
**characterised in that** the clamp limbs (2) have a smaller thickness in the region of the clamp jaws (15) than in the region of their attachment end (21).

10. Medical clamp (1) according to one of claims 1 to 9,
**characterised in that** the thickness of the clamp limbs (2) decreases continuously in a transition region (22) in the direction of the clamp jaws (15).

11. Medical clamp (1) according to one of claims 1 to 10,
**characterised in that** the clamp limbs (46) feature L-shaped clamp jaws (47).

12. Medical clamp (1) according to claim 11,
**characterised in that** the L-shaped clamp jaws (47) are at an angle of approximately 20° to 90°, but preferably an angle of approximately 40° to 70°, to the central longitudinal axis (48) of the clamp limbs (46).

13. Medical clamp (1) according to one of claims 1 to 10,
**characterised in that** the clamp limbs (36) feature Y-shaped clamp jaws (37).

14. Medical clamp (1) according to claim 13,
**characterised in that** the clamp arms (38) of the Y-shaped clamp jaws (37) are preferably angled symmetrically to the central longitudinal axis (39) of the clamp limbs (36) and are at an angle of approximately 55° to 75°, but preferably an angle of approximately 60° to 70°, to one another.

15. Medical clamp (1) according to one of claims 1 to 14,
**characterised in that** the covering devices (24) of the clamp limbs (2) can be actuated independently of one another.

16. Medical clamp (1) according to one of claims 1 to 15,
**characterised in that** the clamp limbs (2) feature clamp jaws (15) with fixing spikes (16) or fixing edges (45).

17. Medical clamp (1) according to one of claims 1 to 16,
**characterised in that** parts of the clamp (1) are made of a non-metal.

18. Medical clamp (1) according to one of claims 1 to 17, **characterised in that** on the grip part (3) an attachment device (53) is provided for a marking element (51), which is configured to determine its position with the aid of a contactless locating method, in particular for use as a reference marking for a positioning system for percutaneous interventions.

19. Medical clamp (1) according to claim 18, having such a marking element (51), **characterised in that** the marking element (51) is disposed away from the central longitudinal axis (57) of the clamp (1).

## Revendications

1. Pince médicale (1), en particulier pince de colonne vertébrale, comprenant deux branches de serrage (2) dotées de mâchoires de serrage (15), les branches de serrage (2) étant reliées à une partie formant poignée (3), la partie formant poignée (3) étant conçue pour exécuter un mouvement de serrage des branches de serrage (2), et les branches de serrage (2) étant reliées de façon désolidarisable à la partie formant poignée (3), **caractérisée en ce qu'**il est associé à chaque branche de serrage (2) un dispositif de recouvrement (24) destiné à recouvrir au moins partiellement les mâchoires de serrage (15), le dispositif de recouvrement (24) étant réalisé sous forme de fourreau (25) de forme tubulaire au moins partiellement ouvert vers un côté, et le dispositif de recouvrement (24) disposé sur la branche de serrage (2) pouvant passer d'une première position, dans laquelle il recouvre au moins partiellement la mâchoire de serrage (15) de la branche de serrage (2), à une deuxième position, dans laquelle il ne recouvre pas la mâchoire de serrage (15) de la branche de serrage (2).

2. Pince médicale (1) selon la revendication 1, **caractérisée en ce que** la partie formant poignée (3) présente une première branche d'actionnement (4) et une deuxième branche d'actionnement (5), lesquelles sont mobiles l'une par rapport à l'autre.

3. Pince médicale (1) selon la revendication 2, **caractérisée en ce que** les deux branches d'actionnement (4, 5) sont articulées entre elles à l'une de leurs extrémités.

4. Pince médicale (1) selon la revendication 2 ou 3, **caractérisée en ce que** les deux branches d'actionnement (4, 5) peuvent se rapprocher et s'éloigner l'une de l'autre par l'intermédiaire d'un pignon central (9).

5. Pince médicale (1) selon la revendication 4, **caractérisée en ce que** le pignon central (9) comporte une broche (12) munie d'un disque moletée (11).

6. Pince médicale (1) selon l'une des revendications 1 à 5, **caractérisée en ce que** les branches de serrage (2) sont reliées à la partie formant poignée (3) par un assemblage par encliquetage, par enclenchement ou par serrage.

7. Pince médicale (1) selon l'une des revendications 1 à 6, **caractérisée en ce que** les branches de serrage (2) sont reliées de façon désolidarisable individuellement à la partie formant poignée (3).

8. Pince médicale (1) selon l'une des revendications 1 à 7, **caractérisée en ce que** les branches de serrage (2) présentent des mâchoires de serrage droites (15).

9. Pince médicale (1) selon l'une des revendications 1 à 8, **caractérisée en ce que** les branches de serrage (2) présentent une moindre épaisseur au niveau des mâchoires de serrage (15) qu'au niveau de leur extrémité de fixation (21).

10. Pince médicale (1) selon l'une des revendications 1 à 9, **caractérisée en ce que** l'épaisseur des branches de serrage (2) diminue en continu en direction des mâchoires de serrage (15) dans une zone de transition (22).

11. Pince médicale (1) selon l'une des revendications 1 à 10, **caractérisée en ce que** les branches de serrage (46) présentent des mâchoires de serrage en L (47).

12. Pince médicale (1) selon la revendication 11, **caractérisée en ce que** les mâchoires de serrage en L (47) renferment avec l'axe longitudinal médian (48) des branches de serrage (46) un angle d'environ 20° à 90°, mais de préférence un angle d'environ 40° à 70°.

13. Pince médicale (1) selon l'une des revendications 1 à 10, **caractérisée en ce que** les branches de serrage (36) présentent des mâchoires de serrage en Y (37).

14. Pince médicale (1) selon la revendication 13, **caractérisée en ce que** les bras de serrage (38) des mâchoires de serrage en Y (37) sont coudés, de préférence symétriquement, par rapport à l'axe longitudinal médian (39) des branches de serrage (36) et renferment entre eux un angle d'environ 55° à 75°, mais de préférence un angle d'environ 60° à 70°.

15. Pince médicale (1) selon l'une des revendications 1 à 14, **caractérisée en ce que** les dispositifs de recouvrement (24) des branches de serrage (2) sont actionnables indépendamment l'un de l'autre.

16. Pince médicale (1) selon l'une des revendications 1 à 15, **caractérisée en ce que** les branches de serrage (2) présentent des mâchoires de serrage (15) dotées de pointes de fixation (16) ou d'arêtes de fixation (45).

17. Pince médicale (1) selon l'une des revendications 1 à 16, **caractérisée en ce que** des parties de la pince (1) sont constituées d'un non-métal.

18. Pince médicale (1) selon l'une des revendications 1 à 17, **caractérisée en ce qu'**il est prévu, sur la partie formant poignée (3), un dispositif de fixation (53) d'un élément de repérage (51) conçu pour déterminer sa position à l'aide d'une méthode de localisation sans contact, en particulier pour être utilisé comme repère de référence d'un système de positionnement pour interventions percutanées.

19. Pince médicale (1) selon la revendication 18 comprenant un tel élément de repérage (51), **caractérisée en ce que** l'élément de repérage (51) est disposé à distance de l'axe longitudinal médian (57) de la pince (1).
